# EUROPÄISCHE PATENTSCHRIFT

(11) **EP 1 012 175 B1**
(45) Veröffentlichungstag und Bekanntmachung des Hinweises auf die Patenterteilung: **08.01.2003**
(21) Anmeldenummer: 98946452.4
(22) Anmeldetag: 12.09.1998
(51) Int. Cl.: C07K 7/06, C07K 7/08, C07K 14/755, G01N 33/68

(54) **PEPTID MIT AFFINITÄT ZU GERINNUNGSFAKTOR VIII**
A PEPTIDE WITH AN AFFINITY FOR CLOTTING FACTOR VIII
PEPTIDE PRESENTANT UNE AFFINITE VIS-A-VIS DU FACTEUR DE COAGULATION VIII

(30) Priorität: 13.09.1997 DE 19740310
(43) Veröffentlichungstag der Anmeldung: 28.06.2000
(73) Patentinhaber: Octapharma AG, 8853 Lachen (CH)
(72) Erfinder: JUNGBAUER, Alois, 1190 Wien (AT); NECINA, Roman, 1020 Wien (AT); JOSIC, Djuro, 1020 Wien (AT)
(74) Vertreter: Meyers, Hans-Wilhelm, Dr.Dipl.-Chem.
(86) Internationale Anmeldenummer: EP9805822
(87) Internationale Veröffentlichungsnummer: WO99014232

(56) Entgegenhaltungen:
- EP-A- 0 294 025
- EP-A- 0 296 645
- WO-A-97/41220
- DE-A- 19 521 313
- J.M. LAVERGNE ET AL: "Primary structure of the factor VIII binding domain of human porcine and rabbit von Willebrand factor" BIOCHEMICAL AND BIOPHYSICAL RESEARCH COMMUNICATIONS., Bd. 194, Nr. 3, 1993, Seiten 1019-1024, XP002092107 ORLANDO, FL US

### SEQUENZPROTOKOLL

(1) ALLGEMEINE ANGABEN:
   (i) ANMELDER:
      (A) NAME: Octapharma AG
      (B) STRASSE: Haus im Park / Postfach
      (C) ORT: Ziegelbruecke
      (E) LAND: Schweiz
      (F) POSTLEITZAHL: 8866
   (ii) BEZEICHNUNG DER ERFINDUNG: Peptid mit Affinitaet zu Gerinnungsfaktor
      VIII
   (iii) ANZAHL DER SEQUENZEN: 94
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 1:
   Gly Cys Val Ser Gly Cys Leu Cys
   1               5
(2) ANGABEN ZU SEQ ID NO: 2:
   (i) SEQUENZ KENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 2:
   Cys Val Ser Gly Cys Leu Cys Pro
   1               5
(2) ANGABEN ZU SEQ ID NO: 3:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 3:
   Val Ser Gly Cys Leu Cys Pro Pro
   1               5
(2) ANGABEN ZU SEQ ID NO: 4:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 4:
   Ser Gly Cys Leu Cys Pro Pro Gly
   1               5
(2) ANGABEN ZU SEQ ID NO: 5:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 5:
   Gly Cys Leu Cys Pro Pro Gly Met
   1               5
(2) ANGABEN ZU SEQ ID NO: 6:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 6:
   Cys Leu Cys Pro Pro Gly Met Val
   1               5
(2) ANGABEN ZU SEQ ID NO: 7:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 7:
   Leu Cys Pro Pro Gly Met Val Arg
   1               5
(2) ANGABEN ZU SEQ ID NO: 8:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 8:
   Cys Pro Pro Gly Met Val Arg His
   1               5
(2) ANGABEN ZU SEQ ID NO: 9:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 9:
   Pro Pro Gly Met Val Arg His Glu
   1               5
(2) ANGABEN ZU SEQ ID NO: 10:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 10:
   Pro Gly Met Val Arg His Glu Asn
   1               5
(2) ANGABEN ZU SEQ ID NO: 11:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 11:
   Gly Met Val Arg His Glu Asn Arg
   1               5
(2) ANGABEN ZU SEQ ID NO: 12:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 12:
   Met Val Arg His Glu Asn Arg Cys
   1               5
(2) ANGABEN ZU SEQ ID NO: 13:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 13:
   Val Arg His Glu Asn Arg Cys Val
   1               5
(2) ANGABEN ZU SEQ ID NO: 14:
   (1) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 14:
   Arg His Glu Asn Arg Cys Val Ala
   1               5
(2) ANGABEN ZU SEQ ID NO: 15:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 15:
   His Glu Asn Arg Cys Val Ala Leu
   1 5
(2) ANGABEN ZU SEQ ID NO: 16:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 16:
   Glu Asn Arg Cys Val Ala Leu Glu
   1 5
(2) ANGABEN ZU SEQ ID NO: 17:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 17:
   Asn Arg Cys Val Ala Leu Glu Arg
   1 5
(2) ANGABEN ZU SEQ ID NO: 18:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 18:
   Arg Cys Val Ala Leu Glu Arg Cys
   1 5
(2) ANGABEN ZU SEQ ID NO: 19:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 19:
   Cys Val Ala Leu Glu Arg Cys Pro
   1               5
(2) ANGABEN ZU SEQ ID NO: 20:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 20:
   Val Ala Leu Glu Arg Cys Pro Cys
   1               5
(2) ANGABEN ZU SEQ ID NO: 21:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 21:
   Ala Leu Glu Arg Cys Pro Cys Phe
   1               5
(2) ANGABEN ZU SEQ ID NO: 22:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 22:
   Leu Glu Arg Cys Pro Cys Phe His
   1               5
(2) ANGABEN ZU SEQ ID NO: 23:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 23:
   Glu Arg Cys Pro Cys Phe His Gln
   1               5
(2) ANGABEN ZU SEQ ID NO: 24:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 24:
   Arg Cys Pro Cys Phe His Gln Gly
   1               5
(2) ANGABEN ZU SEQ ID NO: 25:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 25:
   Cys Pro Cys Phe His Gln Gly Lys
   1               5
(2) ANGABEN ZU SEQ ID NO: 26:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 26:
   Pro Cys Phe His Gln Gly Lys Glu
   1               5
(2) ANGABEN ZU SEQ ID NO: 27:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 27:
   Cys Phe His Gln Gly Lys Glu Tyr
   1               5
(2) ANGABEN ZU SEQ ID NO: 28:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 28:
   Phe His Gln Gly Lys Glu Tyr Ala
   1               5
(2) ANGABEN ZU SEQ ID NO: 29:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 29:
   His Gln Gly Lys Glu Tyr Ala Pro
   1               5
(2) ANGABEN ZU SEQ ID NO: 30:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 30:
   Gln Gly Lys Glu Tyr Ala Pro Gly
   1               5
(2) ANGABEN ZU SEQ ID NO: 31:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 31:
   Gly Lys Glu Tyr Ala Pro Gly Glu
   1               5
(2) ANGABEN ZU SEQ ID NO: 32:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 32:
   Lys Glu Tyr Ala Pro Gly Glu Thr
   1               5
(2) ANGABEN ZU SEQ ID NO: 33:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 33:
   Glu Tyr Ala Pro Gly Glu Thr Val
   1               5
(2) ANGABEN ZU SEQ ID NO: 34:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 34:
   Tyr Ala Pro Gly Glu Thr Val Lys
   1               5
(2) ANGABEN ZU SEQ ID NO: 35:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 35:
   Ala Pro Gly Glu Thr Val Lys Ile
   1               5
(2) ANGABEN ZU SEQ ID NO: 36:
   (i) SEQUENZKEMNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 36:
   Pro Gly Glu Thr Val Lys Ile Gly
   1               5
(2) ANGABEN ZU SEQ ID NO: 37:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 37:
   Gly Glu Thr Val Lys Ile Gly Cys
   1               5
(2) ANGABEN ZU SEQ ID NO: 38:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 38:
   Glu Thr Val Lys Ile Gly Cys Asn
   1               5
(2) ANGABEN ZU SEQ ID NO: 39:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 39:
   Thr Val Lys Ile Gly Cys Asn Thr
   1               5
(2) ANGABEN ZU SEQ ID NO: 40:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 40:
   Val Lys Ile Gly Cys Asn Thr Cys
   1               5
(2) ANGABEN ZU SEQ ID NO: 41:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 41:
   Lys Ile Gly Cys Asn Thr Cys Val
   1               5
(2) ANGABEN ZU SEQ ID NO: 42:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 42:
   Ile Gly Cys Asn Thr Cys Val Cys
   1               5
(2) ANGABEN ZU SEQ ID NO: 43:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 43:
   Gly Cys Asn Thr Cys Val Cys Arg
   1               5
(2) ANGABEN ZU SEQ ID NO: 44:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 44:
   Cys Asn Thr Cys Val Cys Arg Asp
   1               5
(2) ANGABEN ZU SEQ ID NO: 45:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi.) SEQUENZBESCHREIBUNG: SEQ ID NO: 45:
(2) ANGABEN ZU SEQ ID NO: 46:
   (i) SEQUENZKENNZEICHEN:
      (A) LÄNGE: 8 Aminosäuren
      (B) ART: Aminosäure
      (C) STRANGFORM: Einzelscrang
      (D) TOPOLOGIE: nicht bekannt
   (ii) ART DES MOLEKÜLS: Peptid
   (xi) SEQUENZBESCHREIBUNG: SEQ ID NO: 46:
   Thr Cys Val Cys Arg Asp Arg Lys
   1               5
(2) ANGABEN ZU SEQ ID NO: 47:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 47:
   Cys Val Cys Arg Asp Arg Lys Trp
   1               5
(2) ANGABEN ZU SEQ ID NO: 48:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 48:
   Val Cys Arg Asp Arg Lys Trp Asn
   1               5
(2) ANGABEN ZU SEQ ID NO: 49:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 49:
   Cys Arg Asp Arg Lys Trp Asn Cys
   1               5
(2) ANGABEN ZU SEQ ID NO: 50:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 50:
   Arg Asp Arg Lys Trp Asn Cys Thr
   1               5
(2) ANGABEN ZU SEQ ID NO: 51:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 51:
   Asp Arg Lys Trp Asn Cys Thr Asp
   1               5
(2) ANGABEN ZU SEQ ID NO: 52:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 52:
   Arg Lys Trp Asn Cys Thr Asp His
   1               5
(2) ANGABEN ZU SEQ ID NO: 53:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 53:
   Lys Trp Asn Cys Thr Asp His Val
   1               5
(2) ANGABEN ZU SEQ ID NO: 54:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 54:
   Trp Asn Cys Thr Asp His Val Cys
   1               5
(2) ANGABEN ZU SEQ ID NO: 55:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 55:
   Val Met Ile Lys Cys Glu Tyr Cys
   1               5
(2) ANGABEN ZU SEQ ID NO: 56:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 56:
   Val Met Ile Lys Cys Glu Glu Phe
   1               5
(2) ANGABEN ZU SEQ ID NO: 57:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 57:
   Val Met Ile Lys Glu Phe Tyr Cys
   1               5
(2) ANGABEN ZU SEQ ID NO: 58:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 58:
   Val Met Ile Lys Glu Phe Glu Phe
   1               5
(2) ANGABEN ZU SEQ ID NO: 59:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 59:
   Val Met Ile Lys Trp Glu Tyr Cys
   1 5
(2) ANGABEN ZU SEQ ID NO: 60:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 60:
   Val Met Ile Lys Trp Glu Glu Phe
   1 5
(2) ANGABEN ZU SEQ ID NO: 61:
   (i) SEQUENZKENNZEICHEN:
      (A) LÄNGE: 8 Aminosäuren
      (B) ART: Aminosäure
      (C) STRANGFORM: Einzeistrang
      (D) TOPOLOGIE: nicht bekannt
   (ii) ART DES MOLEKÜLS: Peptid
   (xi) SEQUENZBESCHREIBUNG: SEQ ID NO: 61:
   Val Met Lys Ser Cys Glu Tyr Cys
   1 5
(2) ANGABEN ZU SEQ ID NO: 62:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 62:
   Val Met Lys Ser Cys Glu Glu Phe
   1 5
(2) ANGABEN ZU SEQ ID NO: 63:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 63:
   Val Met Lys Ser Glu Phe Tyr Cys
   1               5
(2) ANGABEN ZU SEQ ID NO: 64:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 64:
   Val Met Lys Ser Glu Phe Glu Phe
   1               5
(2) ANGABEN ZU SEQ ID NO: 65:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 65:
   Val Met Lys Ser Trp Glu Tyr Cys
   1               5
(2) ANGABEN ZU SEQ ID NO: 66:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 66:
   Val Met Lys Ser Trp Glu Glu Phe
   1 5
(2) ANGABEN ZU SEQ ID NO: 67:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 67:
   Glu Trp Ile Lys Cys Glu Tyr Cys
   1 5
(2) ANGABEN ZU SEQ ID NO: 68:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 68:
   Glu Trp Ile Lys Cys Glu Glu Phe
   1 5
(2) ANGABEN ZU SEQ ID NO: 69:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 69:
   Glu Trp Ile Lys Glu Phe Tyr Cys
   1 5
(2) ANGABEN ZU SEQ ID NO: 70:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 70:
   Glu Trp Ile Lys Glu Phe Glu Phe
   1               5
(2) ANGABEN ZU SEQ ID NO: 71:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 71:
   Glu Trp Ile Lys Trp Glu Tyr Cys
   1               5
(2) ANGABEN ZU SEQ ID NO: 72:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 72:
   Glu Trp Ile Lys Trp Glu Glu Phe
   1               5
(2) ANGABEN ZU SEQ ID NO: 73:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 73:
   Glu Trp Lys Ser Cys Glu Tyr Cys
   1               5
(2) ANGABEN ZU SEQ ID NO: 74:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 74:
   Glu Trp Lys Ser Cys Glu Glu Phe
   1               5
(2) ANGABEN ZU SEQ ID NO: 75:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 75:
   Glu Trp Lys Ser Glu Phe Tyr Cys
   1               5
(2) ANGABEN ZU SEQ ID NO: 76:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 76:
   Glu Trp Lys Ser Glu Phe Glu Phe
   1 5
(2) ANGABEN ZU SEQ ID NO: 77:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 77:
   Glu Trp Lys Ser Trp Glu Tyr Cys
   1 5
(2) ANGABEN ZU SEQ ID NO: 78:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 78:
   Glu Trp Lys Ser Trp Glu Glu Phe
   1 5
(2) ANGABEN ZU SEQ ID NO: 79:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 79:
   Glu Tyr Ile Lys Cys Glu Tyr Cys
   1 5
(2) ANGABEN ZU SEQ ID NO: 80:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 80:
   Glu Tyr Ile Lys Cys Glu Glu Phe
   1               5
(2) ANGABEN ZU SEQ ID NO: 81:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 81:
   Glu Tyr Ile Lys Glu Phe Tyr Cys
   1               5
(2) ANGABEN ZU SEQ ID NO: 82:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 82:
   Glu Tyr Ile Lys Glu Phe Glu Phe
   1               5
(2) ANGABEN ZU SEQ ID NO: 83:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 83:
   Glu Tyr Ile Lys Trp Glu Tyr Cys
   1               5
(2) ANGABEN ZU SEQ ID NO: 84:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 84:
   Glu Tyr Ile Lys Trp Glu Glu Phe
   1               5
(2) ANGABEN ZU SEQ ID NO: 85:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 85:
   Glu Tyr Lys Ser Cys Glu Tyr Cys
   1               5
(2) ANGABEN ZU SEQ ID NO: 86:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 86:
   Glu Tyr Lys Ser Cys Glu Glu Phe
   1               5
(2) ANGABEN ZU SEQ ID NO: 87:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 87:
   Glu Tyr Lys Ser Glu Phe Tyr Cys
   1               5
(2) ANGABEN ZU SEQ ID NO: 88:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 88:
   Glu Tyr Lys Ser Glu Phe Glu Phe
   1               5
(2) ANGABEN ZU SEQ ID NO: 89:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 89:
   Glu Tyr Lys Ser Trp Glu Tyr Cys
   1               5
(2) ANGABEN ZU SEQ ID NO: 90:
   (i) SEQUENZKENNZEICHEN:
      (A) LÄNGE: 8 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 90:
   Glu Tyr Lys Ser Trp Glu Glu Phe
   1               5
(2) ANGABEN ZU SEQ ID NO: 91:
   (i) SEQUENZKENNZEICHEN:
      (A) LÄNGE: 10 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 91:
   Leu Cys Pro Pro Gly Met Val Arg His Glu
   1               5                   10
(2) ANGABEN ZU SEQ ID NO: 92:
   (i) SEQUENZKENNZEICHEN:
      (A) LÄNGE: 9 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 92:
   Arg Cys Pro Cys Phe His Gln Gly Lys
   1               5
(2) ANGABEN ZU SEQ ID NO: 93:
   (i) SEQUENZKENNZEICHEN:
      (A) LÄNGE: 9 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 93:
   Cys Phe His Gln Gly Lys Glu Tyr Ala
   1               5
(2) ANGABEN ZU SEQ ID NO: 94:
   (i) SEQUENZKENNZEICHEN:
      (A) LÄNGE: 12 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 94:
   Arg Asp Arg Lys Trp Asn Cys Thr Asp His Val Cys
   1               5                   10

## Beschreibung

Gegenstand der vorliegenden Erfindung ist ein Peptid mit Bindungsaffinität zu Faktor VIII, ein Trägermaterial, an das das erfindungsgemäße Peptid gebunden ist, ein Diagnostikmittel enthaltend das erfindungsgemäße Peptid, Verwendung des Peptids zur Markierung, Identifizierung oder Reinigung von Faktor VIII sowie ein Verfahren zur Herstellung des erfindungsgemäßen Peptids, das in einem Flüssig- oder Festphasensystem synthetisiert wurde.

Eine im Stand der Technik bestens etablierte Methode zur Reinigung von insbesondere komplexen Strukturen wie Proteine stellt die Affinitätschromatographie dar (Yannis Chonis; Process Affinity Chromatography, in Separation Processes in Biotechnology, Ed. J.A. Asenjo, pages 401 to 494, Marcel Dekker, New York 1990; S.R. Narayanon, Preparative affinity chromatography of proteins, J. Chromatogr. A, 658 (1994) 237 to 258). Huang, Ping Y. et al. beschreiben in "Bioorganic & Medicinal Chemistry", Vol. 4, No. 5, pp. 699 bis 708, 1996 die Verwendung von Peptiden, die zur Affinitätsreinigung von von Willebrand Faktor eingesetzt werden können. Der von Willebrand Faktor ist eine Komponente im Blutplasma und spielt bei der Stabilisierung von Faktor VIII eine wichtige Rolle.

Auch Faktor VIII selbst ist ein wertvoller Plasmabestandteil, der insbesondere therapeutisches Interesse besitzt, nämlich zur Behebung von Störungen der Blutgerinnungskaskade, bei der Faktor VIII eine wichtige Rolle spielt. Neben der Faktor VIII Gewinnung mittels rekombinanter Methoden spielt nach wie vor die Präparation von Faktor VIII aus Blutplasma eine wichtige Rolle.

Es sind zahlreiche Methoden zur Reinigung von Faktor VIII mittels chromatographischer Methoden bekannt. Dabei werden auch Methoden der Immunaffinitäts-Chromatographie verwendet, wobei Antikörper gegen Faktor VIII auf einem Träger immobilisiert werden und Faktor VIII haltige Proben mit diesem Trägermaterial in Kontakt gebracht werden, so daß der Faktor VIII durch Immunaffinitätsreaktionen aus den betreffenden Proben isoliert werden kann.

Nachteilig bei der letztgenannten Methode ist aber insbesondere, daß es sich bei den Antikörpern um relativ große Moleküle handelt, die einen Großteil der Oberfläche des Trägermaterials belegen und somit eine Kapazitätsbegrenzung bewirken. Zum anderen ist es nicht trivial, die Antikörper an der Oberfläche dieses Materials zu binden unter Erhalt und Präsentation der spezifischen Bindungsstellen, so daß bei der Immobilisierung von Antikörpern darüber hinaus Kapazität eingebüßt wird durch Inaktivierung des Affinitätsliganden. Desweiteren können Kontaminationen durch Ausbluten der Antikörper aus der Trennsäule erfolgen.

J. M. Lavergne et al.: Biochemical and Biophysical Research Communications, Band 194, Nr. 3, 1993, Seiten 1019 bis 1024, Orlando, FL, US offenbart die Bindungsdomaine des von Willebrand-Faktors, welcher dafür bekannt ist, dass er an den Faktor VIII bindet.

EP-A-0 294 025 offenbart Peptide, welche Fragmente des vWF darstellen und Affinität zu Faktor VIII besitzen.

Aus DE-A-195 21 313 geht ein Verfahren zur affinitätschromatographischen Reinigung von Faktor VIII unter Verwendung von vWF oder einem Derivat davon hervor.

Ziel der vorliegenden Erfindung ist es ein Material bereitzustellen, mit dem eine Reinigung des Faktor VIII erleichtert wird.

Erfindungsgemäß wird dieses Ziel erreicht durch Peptide, welche die Merkmale des Patentanspruchs 1 aufweisen.

Die erfindungsgemäßen Peptide besitzen die Struktur R¹-R³-R⁴-R⁵-R⁶-R⁷-R⁸-R⁹-R¹⁰-R², wobei R³ = V oder E, R⁴ = M, W oder Y, R⁵ = I oder K, R⁶ = K oder S, R⁷ = C, E oder W, R⁸ = E oder F, R⁹ = Y oder E, R¹⁰ = C oder F ist und R¹ und R² die angegebene Bedeutung besitzen. Die letztgenannten Peptide binden an humanen bzw. rekombinanten Faktor VIII.

In dem erfindungsgemäßen Peptid deutet R¹ also das N-terminale Ende des Peptids an, wohingegen R² das Carboxyl-Ende des Peptids andeutet. Es ist für den Fachmann unmittelbar verständlich, daß mit den Strukturelementen auch die diesbezüglichen Salze sowie Derivate wie N-modifizierte Derivate oder Carboxy-terminal modifizierte Derivate gemeint sind.

Als Aminosäuren, die zur Bildung des erfindungsgemäßen Peptids herangezogen werden können, gehören sowohl die natürlich vorkommenden Aminosäuren wie auch nicht proteinogene Aminosäuren. Es handelt sich dabei regelmäßig um α-Aminosäuren. Als Strukturvarianten der Aminosäuren kommen solche In Betracht, die in der Seitenkette Modifikationen aufweisen, zyklische Peptide, verzweigte oder mixotrope Peptide.

Das erfindungsgemäße Peptid weist zwischen dem N- bzw. L-Terminus mindestens drei Aminosäuren, die peptidisch verknüpft sind, auf, das Strukturelement zwischen dem N- und C-Terminus ist ein Octapeptid, welches sich durch Bindung zum Faktor VIII auszeichnet. Die Octapeptide der angegebenen Struktur gemäß Seq. ID. No. 1 bis 94 sind in der Lage, Faktor VIII spezifisch zu binden.

Bevorzugt sind Peptide, bei denen R⁷ = W, R⁸ = E, R⁹ = E oder Y, R¹⁰ = F oder C sind und R¹, R² sowie R³ bis R⁶ die angegebene Bedeutung haben. Hiervon werden die erfindungsgemäßen Peptide mit den Seq. Id. No. 66, 72, 78, 84, 89 und 90 erfaßt. Die letztgenannten Peptide besitzen eine auffallende Affinität sowohl zu rekombinantem Faktor VIII wie Faktor VIII, der aus Plasma hergestellt wurde.

Die Octapeptide mit den Seq. Id. No. 61, 66, 71, 72, 76, 77, 78, 81 bis 84, 89 und 90 zeichnen sich durch Bindung zu pdFVIII (Faktor VIII aus Plasma) isoliert aus. Dabei bindet das Peptid mit der Seq. Id. No. 61 keinen rekombinanten Faktor VIII. Die Octapeptide mit den Seq. Id. No. 56 bis 58, 66, 68, 71, 72, 76 bis 78, 81 bis 84, 87 bis 90 binden rekombinanten Faktor VIII, wobei die Octapeptide mit den Seq. Id. No. 66, 71, 72, 76 bis 78, 81 bis 84 und 89 und 90 auch aus Plasma isolierten Faktor VIII binden. Erfindungsgemäß insbesondere bevorzugt sind die Octapeptide mit den Seq. Id. No. 66, 72, 78, 84, 89 und 90.

Die Peptide mit den Seq. Id. No. 66 und 90 bis 94 eignen sich insbesondere für die Affinitätschromatographie von rFVIII (rekombinantem Faktor VIII) und die Peptide 66, 72 sowie 91 bis 94 für die Affinitätschromatographie von pdFVIII.

Erfindungsgemäß können diese Peptide auch zur Herstellung eines erfindungsgemäßen Trägermaterials dienen. Dieses Trägermaterial weist eine Oberfläche mit daran gebundenen Peptiden gemäß der Erfindung auf und ist Gegenstand der Ansprüche 8 bis 9.

Die erfindungsgemäßen Peptide werden an der Oberfläche des erfindungsgemäßen Trägermaterials, vorzugsweise chemisch gebunden. Unter chemischer Bindung werden sowohl kovalente als auch ionische, hydrophobe und/oder komplexe Wechselwirkungen verstanden.

Das Trägermaterial besteht vorzugsweise aus anorganischem oder organischem, insbesondere polymerem Material. Dazu können die an sich in der Chromatographie von Biopolymeren üblicherweise einsetzbaren polymeren Materialien verwendet werden.

Die erfindungsgemäßen Peptide werden vorzugsweise durch Spacer (Arme) an die Oberfläche des Trägermaterials gebunden. Diese Spacer sind dem Fachmann bekannt. Die Spacer sind vorzugsweise so beschaffen, daß sie im chromatographischen Trennprozeß die Affinitätsbindung zwischen Faktor VIII und den Peptiden nicht stören.

Gewöhnlich bestehen Spacer aus linearen, niedermolekularen Kohlenwasserstoffketten, die an beiden Enden mit funktionellen Gruppen ausgestattet sind. Eine Zusammenfassung des Standes der Technik findet man bei G.T. Hermanson, A.K..Mallia und P.K. Smith; Immobilized Affinity Ligand Techniques, Academic Press, Inc. San Diego, New York, Boston, London, Sydney, Tokyo, Toronto 1992. Ein Ende wird an einen festen Träger gebunden, während das andere Ende zur Bindung des Peptidliganden dient. Die Kohlenwasserstoffketten können desweiteren modifiziert sein. Insbesondere kommen als Spacer Diaminodipropylamin (DADPA) (3,3'-Iminobispropylamin), Bernsteinsäure (als Anhydrid), 6-Aminocapronsäure, 1,3-Diamino-2-propanol, 1,6-Diaminohexan (DAH) und/oder Ethylendiamin (EDA) in Betracht. Desweiteren können auch Aminosäuren, Peptide, Zucker und andere Polyole als Spacer fungieren. Ebenfalls kommt Iminodiessigsäure oder Lysin als Spacer in Betracht. Lysin wird beispielsweise als Fmoc-Lys (Fmoc)-OH oder Fmoc-(Lys)-(Boc)-OH an einen Träger gebunden. Durch die Einführung der Schutzgruppen wird Lysin mit Carboxylterminus an den Träger gebunden. Durch selektive Abspaltung der Schutzgruppen erhält man freie Aminogruppen, an denen mindestens ein Peptid gebunden werden kann. Die Aminogruppen des Lysin können auch durch andere Schutzgruppen blockiert werden.

Als chromatographische Träger sind insbesondere Polymere mit hydrophiler Oberfläche, z.B. sogenannte Tentakel-Materialien einsetzbar. Auch die Immobilisierung der erfindungsgemäßen Peptide an kompakte poröse Disks und/oder Membranen, jeweils vorzugsweise mit hydrophiler Oberfläche, ist vorteilhaft. Entsprechende Vorrichtungen sind in der WO-A-96/06158 beschrieben.

Aufgrund der Tatsache, daß die erfindungsgemäßen Peptide mit Faktor VIII in Wechselwirkung treten, sind die erfindungsgemäßen Peptid-Verbindungen als Diagnostikmittel für Faktor VIII geeignet. Das erfindungsgemäße Diagnostikmittel enthält mithin mindestens ein erfindungsgemäßes Peptid, oder ein erfindungsgemäßes Trägermaterial, an das mindestens ein erfindungsgemäßes Peptid gebunden ist sowie gegebenenfalls Hilfsmittel.

Zur Kenntlichmachung einer erfolgten Reaktion eines erfindungsgemäßen Peptids mit Faktor VIII ist das erfindungsgemäße Peptid markiert. Als Markierung kann beispielsweise eine radioaktive Markierung dienen oder eine Markierung mittels Fluoreszenzliganden, das Avidin/Streptavidin-System oder Enzyme, die Farbreaktionen hervorrufen können, wie sie in der ELISA-Technik allgemein bekannt sind.

Die erfindungsgemäßen Peptide sind zur Markierung, Identifizierung oder Reinigung von Faktor VIII geeignet.

Die erfindungsgemäßen Peptide lassen sich nach in der Peptidchemie gut etablierten Methoden herstellen, insbesondere nach der Merrifield-Festphasensynthese oder in flüssiger Phase.

### Beispiele:

### Beispiel 1

### Affinitätschromatographie

Aus Membranen, auf die die jeweiligen Peptide immobilisiert wurden, wurden jeweils 30 Scheiben mit einem Durchmesser von 11 mm ausgestanzt. Diese wurden in eine HR 10 Säule (Pharmacia Biotechnology, Uppsala, Schweden) gepackt und zwischen zwei Stempeln fixiert.

Für alle chromatographischen Experimente wurde das Chromatographiesystem ProSys (Biosepra Inc. Marlborough, USA) verwendet.

PBS Puffer pH 7.0 wurde als Äquilibrierungs- und Waschpuffer verwendet. Für die Elution wurde ein 0,1 M Glycinpuffer pH 3.0 angewandt. Die Flußgeschwindigkeit betrug bei allen Versuchen 0,1 ml/min.

### pdFVII

Lyophilisierter Faktor VIII aus Plasma wurde in 11 ml destilliertem Wasser zu einer Konzentration von 90 Units/ml aufgelöst. Davon wurden jeweils 4 ml mit einer Flußrate von 0,1 ml/min aufgetragen. Anschließend wurden die Affinitätsträger mit PBS-Puffer pH 7.0 gewaschen bis das UV Signal (280 nm) wieder die Basislinie erreichte. Das gebundene Material wurde mit 0,1 M Glycin pH 3.0 eluiert. Die gesammelten Fraktionen wurde mittels SDS-Page unter reduzierenden Bedingungen und Western Blot analysiert. Auf diese Weise wurden Peptide und der ßAla-ßAla-Blank auf ihre Fähigkeit Faktor VIII aus dem Octavipräparat zu binden untersucht.

### rFVIII

Die Bindung von rekombinantem Faktor VIII wurde mittels Kogenate® (Firma Bayer) untersucht. Jeweils 400 µl des Präparates (200 Units) wurden mit 1.400 µl destilliertem Wasser verdünnt und anschließend mit einer Flußrate von 0,1 ml/min auf den Affinitätsträger aufgetragen. Das Waschen erfolgte wiederum mit PBS, pH 7.0 und die darauf folgende Elution mit 0,1 M Glycin, pH 3.0. Die Fraktionen wurden mittels SDS-Page unter reduzierenden Bedingungen und Western-Blot analysiert.

### SDS-PAGE

Der Durchlauf und die Eluate aller Läufe wurden gesammelt und mittels SDS-PAGE unter reduzierenden Bedingungen und Western-Blot analysiert.

Für die Auftrennung wurden 4 bis 20% Tris-Glycin Gele von Novex verwendet. Die Proben wurden mit SDS-Probepuffer (1,5 M Tris HCl pH 8.45, 1,2 ml Glycerin, 0,4 g SDS, 0,1% Coomassie Blue, 0,1% Phenol Rot pro 10 ml), und 5% ß-Mercaptoethanol versetzt und über Nacht bei 4°C inkubiert. Nach der Trennung wurden die Gele silbergefärbt und eingescannt.

### Western Blot

Nach Beendigung der elektrophoretischen Trennung wurden die Gele auf eine Nitrocellulose Membran bei 200 mA konstant für zwei Stunden elektrogeblottet. Anschließend wurde die Membran mit PBS + 3% BSA für zwei Stunden blockiert. Nach Waschen mit PBS Puffer erfolgte die Zugabe der Anti-FVIII Antikörpermischung (Chemicon International Inc., MAB038, Sera-Lab MAS 530, MAS 531, MAS 532) für zwei Stunden. Nach Waschen mit PBS wurde mit Anti Maus IgG alkalische Phosphatase (1:1000, Sigma Chemicals, A-3438) für eine Stunde inkubiert. Die Entwicklung erfolgte mit 0,1 M Tris, pH 9.2, NBT und BCIP.

Die Peptide Seq. Id. No. 55 bis 90 wurden auf einer Membran immobilisiert. Nach Äquilibrieren mit PBS Puffer pH 7.0 wurde eine Membran mit Faktor VIII Octavi® (240 Units in 10 ml PBS pH 7.0 mit 1% BSA), eine andere mit Kogenate® (250 U in 10 ml PBS, pH 7.0 mit 1% BSA) und eine nur mit PBS, pH 7.0 mit 1% BSA für zwei Stunden inkubiert. Die mit den Peptiden besetzten Membranen wurden sorgfältig mit PBS Puffer pH 7.0 gewaschen und anschließend mit den Anti-FVIII Antikörpern (Chemicon International Inc., MAB038, Sera-Lab MAS 530, MAS 531, MAS 532) für eine Stunde versetzt. Nach Waschen und Inkubieren mit Anti-Maus-IgG/alkalische Phosphatase Konjugat (1:1000, Sigma Chemicals, A-4338) wurde mit Hilfe von NBT und BCIP in 0,1 M Trispuffer, pH 9,2, entwickelt. Faktor VIII bindende Peptide erschienen als gefärbte Spots. Die Kontrollmembran (nur mit PBS pH 7.0 ohne Faktor VIII) wurde mit den beiden anderen verglichen.

### Beispiel 2

### Affinitätschromatographie mit den Peptiden

Die Affinitätschromatographie wurde wie im Beispiel 1 beschrieben, durchgeführt.

### Affinitätschromatographie mit pdFVIII

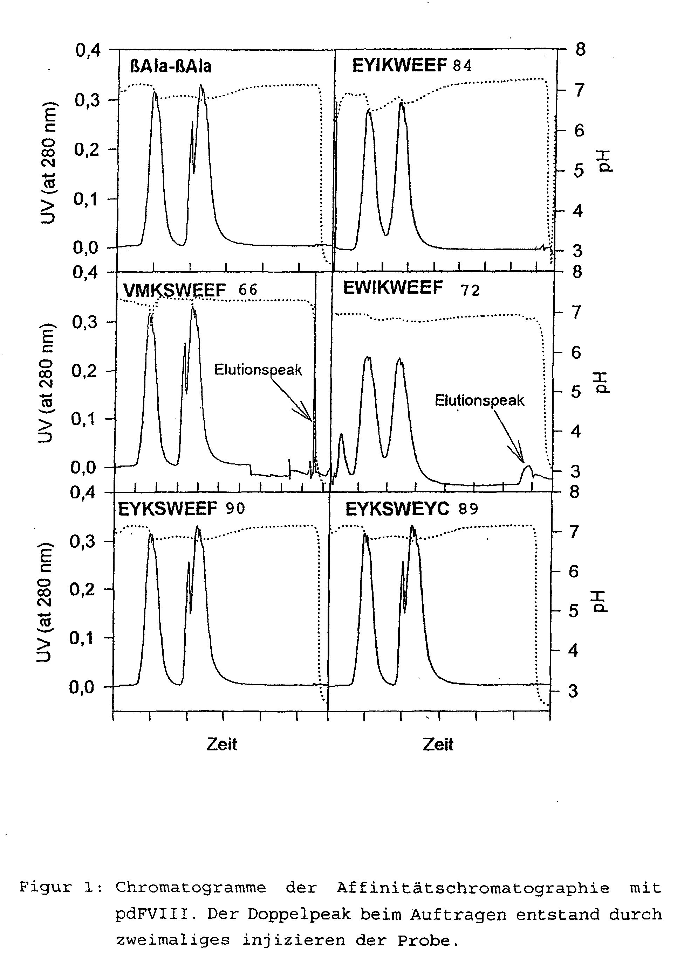

Bei Verwendung der (βAla)₂-Kontrollmembran kann nur ein sehr kleiner Elutionspeak detektiert werden (siehe Figur 1). Auch bei der Silberfärbung der SDS-PAGE unter reduzierenden Bedingungen treten nur sehr schwach gefärbte Banden auf. Der Western Blot des Eluates ist schwach positiv.

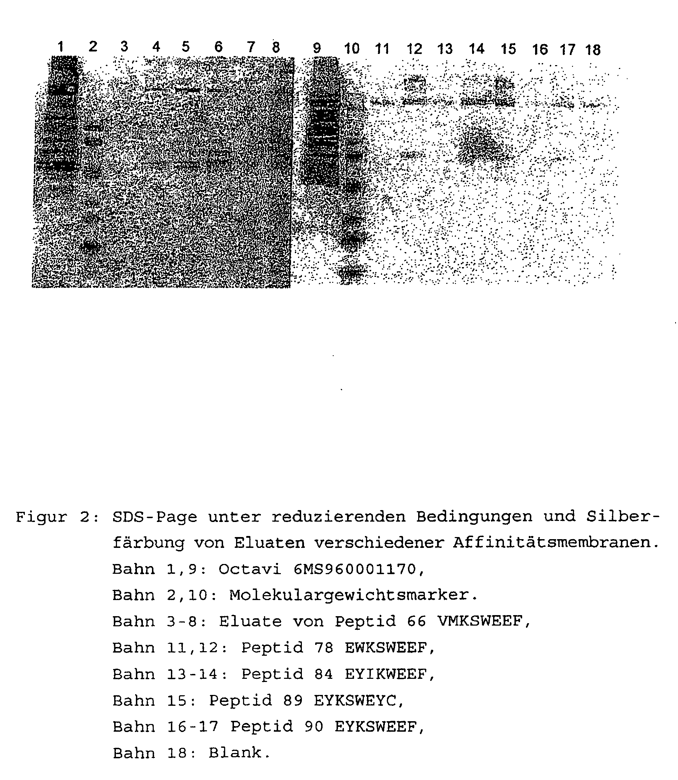

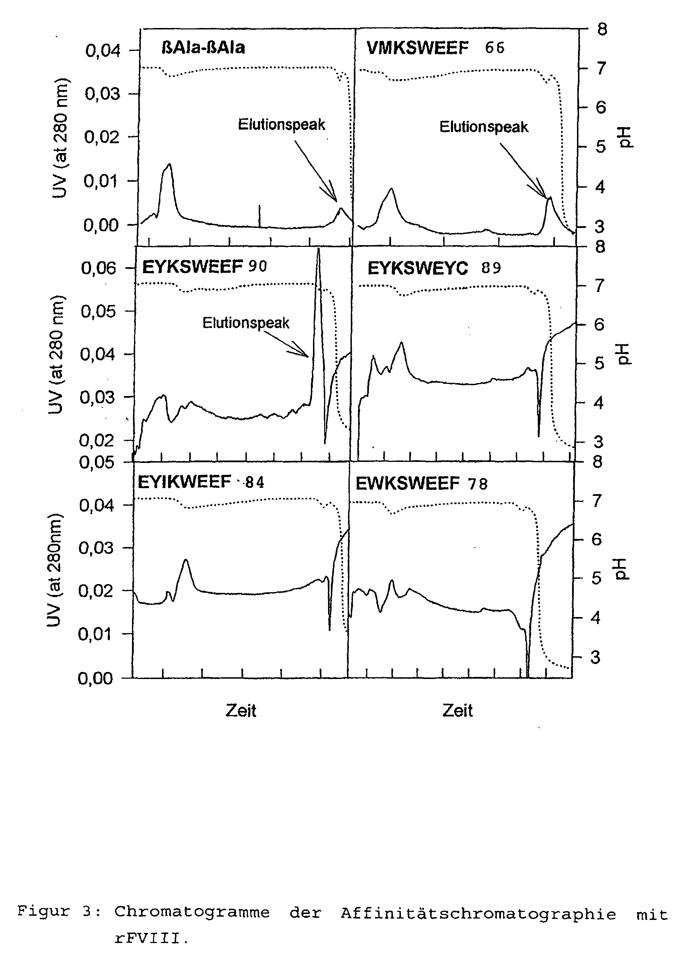

Die Peptide Seq. Id. No. 66 (VMKSWEEF) und 72 (EWIKWEEF) (Figur 2) zeigen größere Elutionspeaks und intensiver gefärbte Banden in der SDS-PAGE unter reduzierenden Bedingungen (siehe Figur 3) und Silberfärbung. In beiden Fällen ist der Western Blot positiv.

### Bindung von rekombinantem Faktor VIII aus Kogenate® von Bayer

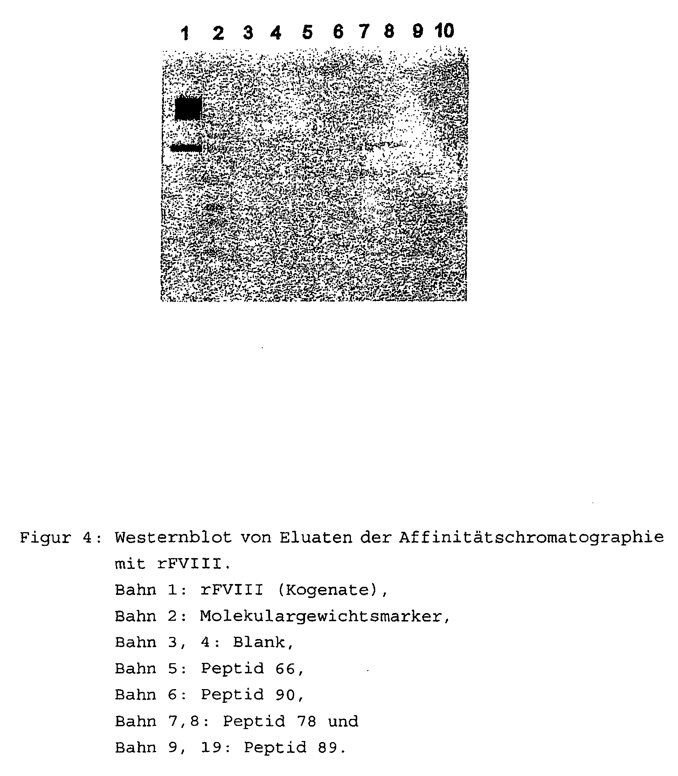

Wie man aus dem Chromatogramm (Figur 4) erkennen kann, bindet die Kontrollmembran, mit ßAla-ßAla immobilisiert, den rekombinanten Faktor VIII nur sehr schwach. Der Western Blot des Eluates fällt positiv aus.

Die Elutionspeaks von den Peptiden VMKSWEEF (Seq. Id. No. 66), EYKSWEEF (Peptid 90) in Figur 4 sind deutlich größer als der der ßAla-ßAla Kontrollmembran. In beiden Fällen ist der Western Blot der Eluate positiv (Figur 4).

## Patentansprüche

1. Peptid der Struktur
R¹ - X - R²,
wobei
R¹ = NH₂ oder ein Peptid ist,
R² = COOH oder ein Peptid und
X = ein mindestens Tripeptid, insbesondere ein Hepta- bis Dodecapeptid ist, das mit Faktor VIII eine Affinitätsbindung eingeht.

2. Peptid nach Anspruch 1, **dadurch gekennzeichnet, daß** X ein Octapeptid bis Dodecapeptid ist.

3. Peptid nach Anspruch 1, wobei
X = -R³-R⁴-R⁵-R⁶-R⁷-R⁸-R⁹-R¹⁰-,
R³ = V oder E,
R⁴ = M, W oder Y,
R⁵ = I oder K,
R⁶ = K oder S,
R⁷ = C, E oder W,
R⁸ = E oder F,
R⁹ = Y oder E,
R¹⁰ = C oder F ist und
R¹ und R² die angegebene Bedeutung besitzen.

4. Peptid nach Anspruch 2, **dadurch gekennzeichnet, daß** X ausgewählt ist aus der Gruppe von Octapeptiden, bestehend aus den Sequenzen mit den Seq. ID. No. 1 bis 94.

5. Peptid nach Anspruch 3, wobei
R⁷ = W,
R⁸ = E,
R⁹ = E oder Y,
R¹⁰ = F oder C ist und
R¹, R² und R³ bis R⁶ die angegebene Bedeutung haben.

6. Peptide nach Anspruch 3 mit der Aminosäuresequenz gemäß Seq. ID. No. 55 bis 90.

7. Peptide nach Anspruch 1 und/oder 2, wobei die Peptide die Seq. ID. No. 91 bis 94 aufweisen.

8. Trägermaterial mit einer Oberfläche, an die Peptide nach mindestens einem der Ansprüche 1 bis 7 gebunden sind.

9. Trägermaterial nach Anspruch 8 aus anorganischem oder organischem, insbesondere polymerem Material.

10. Trägermaterial nach Anspruch 8 und/oder 9, wobei das Peptid oder die Peptide mittels Spacern an der Oberfläche des Trägermaterials gebunden sind.

11. Diagnostikmittel enthaltend mindestens ein Peptid nach mindestens einem der Ansprüche 1 und/oder 7, ein Trägermaterial nach mindestens einem der Ansprüche 8 bis 10 und gegebenenfalls Hilfsmittel sowie gegebenenfalls mindestens ein Peptid in markierter Form.

12. Verwendung der Peptide nach mindestens einem der Ansprüche 1 bis 7 zur Markierung, Identifizierung oder Reinigung von Faktor VIII.

13. Verfahren zur Herstellung der Peptide nach mindestens einem der Ansprüche 1 bis 7 durch Flüssig- oder Festphasensynthese.

## Claims

1. A peptide having the structure
R¹ - X - R²,
wherein
R¹ = NH₂ or a peptide;
R² = COOH or a peptide; and
X = a peptide which is at least a tripeptide, preferably a hepta- to dodecapeptide, which undergoes affinity binding to factor VIII.

2. The peptide according to claim 1, **characterized in that** X is an octapeptide to dodecapeptide.

3. The peptide according to claim 1, wherein
X = -R³-R⁴-R⁵-R⁶-R⁷-R⁸-R⁹-R¹⁰-;
R³ = V or E;
R⁴ = M, W or Y;
R⁵ = I or K;
R⁶ = K or S;
R⁷ = C, E or W;
R⁸ = E or F;
R⁹ = Y or E;
R¹⁰ = C or F; and
R¹ and R² have the indicated meanings.

4. The peptide according to claim 2, **characterized in that** X is selected from the group of octapeptides consisting of the sequences having the SEQ ID NOS. 1 to 94.

5. The peptide according to claim 3, wherein
R⁷ = W;
R⁸ = E;
R⁹ = E or Y;
R¹⁰ = F or C; and
R¹, R² and R³ to R⁶ have the indicated meanings.

6. The peptide according to claim 3 having the amino acid sequence according to SEQ ID NOS. 55 to 90.

7. The peptide according to claim 1 and/or 2 having the SEQ ID NOS. 91 to 94.

8. A support material having a surface to which peptides according to at least one of claims 1 to 7 are bound.

9. The support material according to claim 8 consisting of inorganic or organic, especially polymeric, material.

10. The support material according to claim 8 and/or 9, wherein said peptide or peptides are bound to the surface of the support material through spacers.

11. A diagnostic agent containing at least one peptide according to at least one of claims 1 and/or 7, a support material according to at least one of claims 8 to 10, and optionally auxiliary agents, and optionally at least one peptide in a labeled form.

12. Use of the peptides according to at least one of claims 1 to 7 for the labeling, identification or purification of factor VIII.

13. A process for the preparation of the peptides according to at least one of claims 1 to 7 by liquid or solid phase synthesis.

## Revendications

1. Peptide de structure
R¹ X - R²,
dans laquelle
R¹ = NH₂ ou est un peptide
R² = COOH ou est un peptide, et
X est au moins un tripeptide, notamment un hepta- à dodécapeptide, qui contracte une liaison d'affinité avec le facteur VIII.

2. Peptide selon le revendication 1, **caractérisé en ce que** X est un octa- à dodécapeptide.

3. Peptide selon la revendication 1, dans lequel
X = -R³-R⁴- R⁵-R⁶- R⁷-R⁸- R⁹-R¹⁰-,
R³ = V ou E,
R⁴ = M, W ou Y,
R⁵ = I ou K,
R⁶ = K ou S,
R⁷ = C, E ou W,
R⁸ = E ou F,
R⁹ = Y ou E,
R¹⁰ = C ou F et
R¹ et R² sont tels qu'il est indiqué ci-dessus.

4. Peptide selon la revendication 2, **caractérisé en ce que** X est choisi dans la classe des octapeptides formée par les séquences ayant les numéros d'identification (Seq.lD.No.) 1 à 94.

5. Peptide selon la revendication 3, dans lequel
R⁷ = W,
R⁸ = E,
R⁹ = E ou Y,
R¹⁰ = F ou C, et
R¹ et R² et la série de R³ à R⁶ ont la signification indiquée ci-dessus.

6. Peptides selon la revendication 3 comportant la séquence d'acides aminés selon une des séquences de numéro d'identification (Seq. ID. No.) 55 à 90.

7. Peptides selon la revendication 1 et/ou 2, les peptides présentant une séquence de numéro d'identification ( Seq. ID. No.) de 91 à 94.

8. Matière de support comportant une surface à laquelle les peptides selon au moins une des revendications de 1 à 7 sont liés.

9. Matière de support selon la revendication 8 constituée de matière minérale ou organique, notamment de matière polymère.

10. Matière de support selon la revendication 8 et/ou 9, dans laquelle le peptide ou les peptides sont liés par l'intermédiaire d'espaceurs (segments intermédiaires) à la surface du matériau de support.

11. Agent de diagnostic contenant au moins un peptide selon au moins une des revendications 1 et/ou 7, une matière de support selon au moins une des revendications de 8 à 10 et éventuellement un produit auxiliaire ainsi qu'éventuellement au moins un peptide sous forme marquée.

12. Utilisation des peptides selon au moins une des revendications de 1 à 7 pour le marquage, l'identification et la purification du facteur VIII.

13. Procédé de préparation des peptides selon au moins une des revendications de 1 à 7 par synthèse en phases liquides ou en phases solides.
